# EUROPEAN PATENT SPECIFICATION

(11) **EP 2 147 675 B1**
(45) Date of publication and mention of the grant of the patent: **12.09.2012**
(21) Application number: 08741789.5
(22) Date of filing: 27.02.2008
(51) Int. Cl.: A61K 33/00, A61P 1/00, A61P 11/06, A61P 17/00, A61P 43/00

(54) **AGENT FOR STIMULATING LYMPHATIC DRAINAGE**
MITTEL ZUR STIMULIERUNG DER LYMPHDRAINAGE
PRODUIT POUR STIMULER LE DRAINAGE LYMPHATIQUE

(30) Priority: 17.05.2007 RU 2007118217
(43) Date of publication of application: 27.01.2010
(73) Proprietor: Pikalov, Mikhail Sergeevich, Moscow 117449 (RU); Pikalov, Aleksandr Mikhailovich, Moscow 117218 (RU)
(72) Inventor: Pikalov, Mikhail Sergeevich, Moscow 117449 (RU); Pikalov, Aleksandr Mikhailovich, Moscow 117218 (RU)
(74) Representative: von Füner, Nicolai
(86) International application number: PCT/RU2008/000104
(87) International publication number: WO 2008/143545

(56) References cited:
- EP-A1- 0 889 007
- WO-A1-2005/084603
- WO-A2-02/096225
- DE-A1- 1 963 706
- FR-A1- 2 659 555
- RU-C1- 2 119 802
- RU-C1- 2 147 863
- US-A1- 2003 035 849

## Description

The invention relates to the chemical-pharmaceutical industry, namely to an agent for stimulating a lymphatic drainage including formation and transportation of lymph.

Under conditions of the total contamination of an organism with anthropogenic products the problem of science-based purification of an organism and above all of a habitat of cells has become today one of the main problems of health preservation of all the population of the planet.

Lymphostimulation promotes elimination of metabolites, various xenobiotics out of tissues through the lymphatic system at their primary detoxication (deactivation) at lymph node level, which contributes to the detoxication of tissues, organs and an organism as a whole.

Organism detoxication is performed by various methods, one of them is the balneal influence on organism tissues and organs which is directed at the invigoration of the organism's internal environment with the help of baths, shower-bath and sauna (V.T.Olifirenko, Water-and-Thermal Cure, M., Medicine, 1986, 288 p.), and all these hydrotherapeutic procedures may be accompanied by the addition of aromatic, mineral, radio-active and other agents. RU 2147863 discloses velvet antler cooking water as lymphatic stimulation agent for use in the form of immersion baths.

Physical lymphatic stimulants (local irritants, massage and curative gymnastics) are also widely used (R.T. Panchenkov and others, Lymphostimulation, M., Medicine, 1986, 240 p.).

The disadvantage of well-known lymphostimulation methods and preparations is that they have a local effect and moreover, the influence itself may produce an irritating effect on a patient.

The technical result of the present invention is an improvement of the natural drainage and detoxication function of an organism.

The object of this invention is to provide an agent for stimulating on the basis of a natural substance, namely processed drinking water.

For that purpose a group of inventions is applied directed at the solution of the object set.

A subject matter of the present invention is an agent for stimulating which is composed of drinking water subjected to baromembranous treatment and having a redox potential from +200 to +343 mV, a total mineralisation from 25 to 130 mg/l and a pH value from 6.9 to 8.3. Preferably its daily consumption dose makes up 1.5 to 2.5 1.

The agent for stimulating may be used in the complex therapy for patients with chronic skin diseases, non-oncological diseases of the gastrointestinal tract and chronic obstructive bronchitis.

A further subject matter is a method of obtaining the agent for stimulating lymphatic drainage according to which water is first brought to the state of drinking water and then is treated in a baromembranous plant, where the water is passed through membranes made of a semi-waterproof material with a hole size 0.0001-0.005 µm; the purified water has the following characteristics:

| | |
|---|---|
| redox potential | from +200 to +343 mV |
| total mineralisation | from 25 to 130 mg/l |
| pH value | from 6.9 to 8.3. |

Furthermore, the purified water may be subjected to additional treatment by saturating it with macro- and microelements.

A still further subject matter is a method of lymphatic drainage stimulation for medical treatment of patients with chronic skin diseases, non-oncological diseases of gastrointestinal tract and chronic obstructive bronchitis with the help of the above agent for stimulation, and the agent is taken 30-40 minutes before and 2-2.5 hours after each food intake, altogether 1.5-2.5 1 a day, the first dose is taken on an empty stomach.

The invention is illustrated by the following examples of obtainment and use of the present agent in experiments on animals and volunteers.

Drinking cold tap water which has been previously purified by municipal services and meets the requirements of sanitary standard SanPiN 2.1.4.1074-01 "Drinking Water" is additionally purified (for example, with the help of a domestic reverse-osmosis filter "Atoll") before consumption. "Atoll" reverse-osmosis systems serve for additional purification of water in domestic conditions in order to use it subsequently for drinking, cooking and other purposes. "Atoll" reverse-osmosis systems remove up to 99.9% of all impurities contained in water and prevent formation of scale in heaters. At the same time, the water while going through the filter gets enriched in oxygen that additionally gives it a pleasant fresh taste. The purification method - reverse osmosis - applied in these systems does not provide using of chemicals and is accomplished by passing the water under pressure through a special membrane.

"Atoll" reverse-osmosis systems are produced in Russia (according to specifications TU3697-009-18261557-03) and the USA (NSF/ANSI Standard 058). Water is passed through membranes made of a semi-waterproof material with a hole size 0.0001-0.005 µm; the purified water has the following characteristics:

| | |
|---|---|
| redox potential | from +200 to +343 mV |
| total mineralisation | from 25 to 130 mg/l |
| pH value | from 6.9 to 8.3. |

Furthermore, the purified water may be subjected to additional treatment by saturating it with macro- and microelements (calcium, magnesium, sodium, sulfur, vanadium, carbon, oxygen; iron, iodine, copper, cobalt, zinc, fluorine, selenium, silicon, silver etc.)

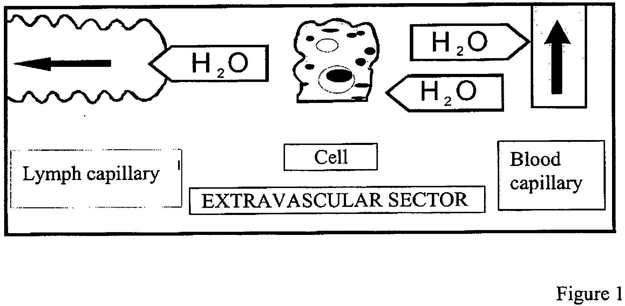

As is well known, lymphatic drainage of tissues (figure 1) is the main way of organism cell habitat purification from metabolic waste excreted by them, coming from blood, and also from toxic substances formed in tissues.

The experiments have been conducted on white common mice and Chinchilla rabbits

### Experiments on mice.

The animals were kept in a special, separate premise with room temperature and daylight. The mice had free access to water and food. All of them were kept in the same conditions as before the experiment. Then the animals were divided into groups (10 individuals in each). One group was given the present water for drinking. The other group of laboratory mice in a series of check experiments was drinking settled tap water during the whole period of time.

After expiration of the designed period of drinking of the present water 0,1% sodium pentobarbital solution was introduced intraperitoneally to the animals on the basis of 0.8 ml to 100 g body weight.

After that a midline laparotomy was performed. A part of the small intestine with a frill were extracted, placed on a special heated table and sprinkled with isotonic solution. The temperature of the sample table and of the isotonic solution was maintained at a physiological level.

A marker (Evans blue) which can be eliminated from the place of introduction only through the lymphatic system was introduced with a syringe in the amount of 0,02 ml in a standard place (ileocecal angle) into a cellular tissue situated along the vessels. The time of mark introduction and excretion was regularly fixed. The time of the dye evacuation into the lymphatic system was estimated which defined the speed of extravascular humoral transportation and lymphatic drainage of tissues.

The data obtained were subjected to statistical analysis. The results obtained were processed with the help of parametric (Student's test) and nonparametric (Mann-Whitney criterion and Dann criterion) methods of assessment. The results with P>95% were considered to be reliable.

The influence of the present water on the lymphatic drainage when used for 5-7 days.

The data of ten experiments in the main group and ten experiments in the check group.

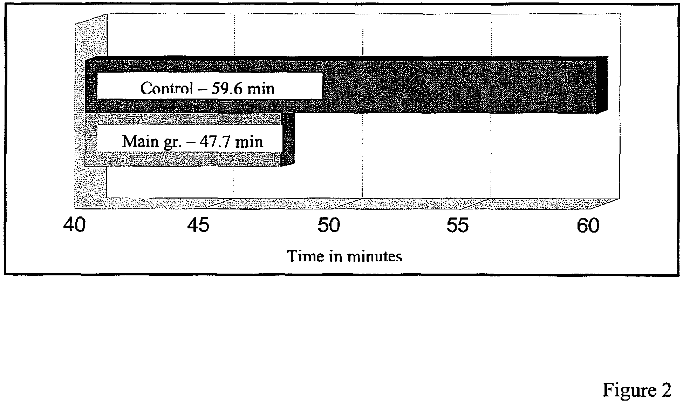

Complete evacuation time of a lymphotropic dye (figure 2):
Main group - 47.7 min.
Check- 59.6 min.
The difference is of high statistical reliability.

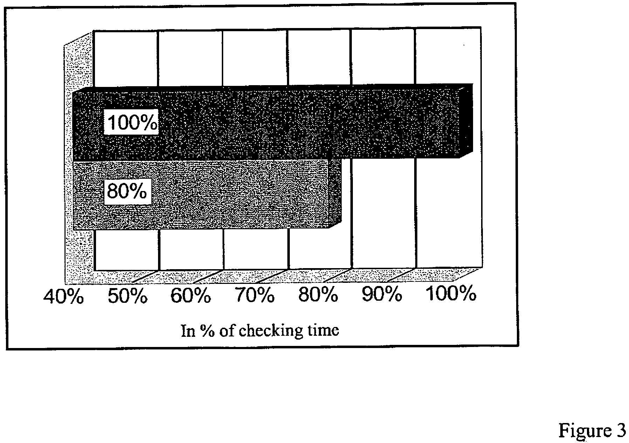

The experiments on mice have ascertained that weekly ingestion of the present water accelerates lymphatic drainage by 20% - 30% (figure 3).

The influence of the present water on the lymphatic drainage when used for 14-17 days.

The data of ten experiments in the main group and ten experiments in the check group have shown identical results with the data obtained when using the water during 5-7 days.

### Experiments on Chinchilla rabbits.

The experiment was conducted on six Chinchilla rabbit males weighing 3 - 3.5 kg.

The present water had been slowly evaporated till the initial quantity reduced three times. Then a concentrated solution of NaCl was added to the remaining solution up to the initial volume with the achievement of its isotonicity.
The following stages were common for all the experiments:
1- narcotisation of the animals by introducing sodium pentobarbital into a marginal. auricular vein in a quantity of 20 mg/kg of the weight;
2- tracheotomy with connection of an artificial pulmonary ventilation apparatus;
3- thoracotomy;
4- thoracic (lymphatic) duct catheterization by the method of A.A. Kornienko and coauthors to determine the lymph efflux rate;
5- femoral vein catheterization to fill up the liquid lost with the lymph, to introduce the examined solution which contained the present water ingredients.

After thoracic duct draining the lymph outflow rate was determined by collecting the lymph into a graduated test-tube during two hours and measuring every 20 minutes the quantity exuded. To maintain the water-salt balance, every 40 minutes an isotonic solution of NaCl was injected intravenously in the amount equal to the lost lymph quantity.

| Volumetric lymph flow from a thoracic duct (ml/min) | |
|---|---|
| Checking of 6 experiments | |
| 1^{st} hour | 0.065±0.004 |
| 2^{nd} hour | 0.067±0.004 |

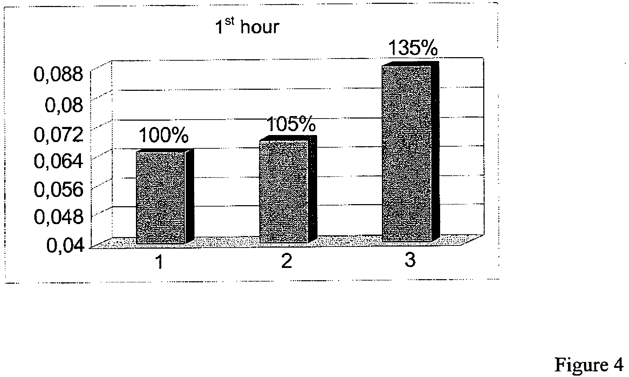

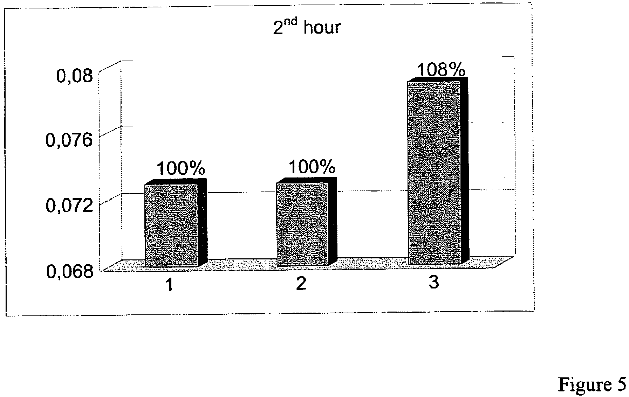

One hour after thoracic duct cutting, an adequate quantity of solution containing the present water ingredients was introduced instead of isotonic solution. The data obtained are reflected in figures 4 and 5. The effect is highly reliable.

| (M± m; n=6) | | | |
|---|---|---|---|
| Before introduction | | After introduction | |
| | | Solution of NaCl (checking) | solution with the present water ingredients |
| 1st hour | 0.065±0.004 | 0.068±0.004 | 0.088±0.005 |
| 2nd hour | 0.073±0.004 | 0.073±0.004 | 0.079±0.004 |

The experiment data show that the present water characteristics stimulate lymph formation.

The ability of the present water to accelerate the lymphatic drainage of tissues was studied on animals.

The administration of the water was included into a complex therapy of 84 patients (the city of Pushchino)

Three series of observations were performed:
1. Chronic locomotor system diseases -28 patients;
2. Skin diseases -28 patients;
3. Gastrointestinal tract pathology -28 patients

They formed 6 groups of 14 patients - three check groups and three main groups. All the groups were comparable in sex, age and time of chronic inflammatory disease.

The first three groups were administered usual boiled water, the remaining three groups took the present water 30-40 minutes before and 2-2.5 hours after each food intake, altogether 1.5-2.5 1 a day, and the first intake was on an empty stomach. The water was additionally saturated with macro- and microelements, but the total mineralisation did not exceed the limits of 25-130 mg/l.

The medical treatment efficiency was estimated by clinical manifestations, biochemical blood analysis results, and ultrasonic scanning data before and after treatment.

The present water included into a complex therapy of chronic locomotor system diseases, skin diseases and gastrointestinal tract pathology has shown an expressed curative effect on patients in comparison with the check groups.

Thus, the above water has a unique stimulating effect on the organism tissue lymphatic drainage, including lymph formation and transportation;

The revealed effect makes it possible to use the present water for endoecological rehabilitation of an organism on the cellular-organismic level owing to 20-30% acceleration of the lymphatic drainage of tissues.

### Explanations to figures 4/5 and 5/5.

Column 1 - checking before introduction;
Column 2 - introduction of NaCl isotonic solution (check 2);
Column 3 - introduction of isotonic solution with present water ingredients.

## Claims

1. An agent for stimulating lymphatic drainage, **characterized in that** it contains drinking water having undergone baromembranous treatment and having a redox potential from +200 to +343 mV, total mineralisation from 25 to 130 mg/l and pH index from 6.9 to 8.3.

2. The agent for stimulating according to claim 1 **characterized in that** the daily dose is 1.5-2.5 1.

3. The agent for stimulating according to claim 1 which can be used in the complex therapy of chronic skin diseases, non-oncological diseases of gastrointestinal tract and chronic obstructive bronchitis.

4. A method of obtaining an agent for stimulating lymphatic drainage **characterized in that** water is first brought to the state of drinking water and then is treated on a baromembranous plant, whereby the water is passed through membranes made of a semipermeable material with a hole size 0.0001-0.005 µm, whereupon the purified water is saturated with macro- and microelements and has the following characteristics:
| | |
|---|---|
| redox potential | from +200 to +343 mV |
| total mineralisation | from 25 to 130 mg/l |
| pH index | from 6.9 to 8.3. |

5. The agent for stimulating according to claim 1 for used in a method of lymphatic drainage stimulation in a medical treatment of patients with chronic skin diseases, non-oncological diseases of gastrointestinal tract and chronic obstructive bronchitis, and wherein the agent is taken 30-40 minutes before and 2-2.5 hours after each meal, altogether 1.5-2.5 1 a day, the first dose is taken during the morning fast.

## Patentansprüche

1. Mittel zur Stimulierung von Lymphdrainage, **dadurch gekennzeichnet, dass** es Trinkwasser nach erfolgter Baromembranbehandlung mit einem Redoxpotential von +200 - + 343 mV, einer Gesamtmineralisation von 25 - 130 mg/l und einem pH-Wert von 6,9 - 8,3 enthält.

2. Mittel zur Stimulierung gemäß Anspruch1, **dadurch gekennzeichnet, dass** die Tagesdosis 1,5 - 2,5 beträgt.

3. Mittel zur Stimulierung gemäß Anspruch 1, das zur Komplextherapie von chronischen Hauterkrankungen, nichtonkologischen Erkrankungen des Verdauungstrakts und chronischer obstruktiver Bronchitis verwendet werden kann.

4. Verfahren zur Herstellung eines Mittels zur Stimulierung von Lymphdrainage, **dadurch gekennzeichnet, dass** Wasser zuerst in den Zustand von Trinkwasser überführt und dann in einer Baromembrananlage behandelt wird, wobei das Wasser durch aus einem semipermeablen Material mit einer Porengröße 0,0001 - 0,005 µm hergestellte Membranen geleitet wird, wonach das gereinigte Wasser mit Makro- und Mikroelementen gesättigt wird und die folgenden Kenndaten aufweist:
| | |
|---|---|
| Redoxpotentioal | +200 - + 343 mV |
| Gesamtmineralisation | 25 - 130 mg/l |
| pH-Wert | 6,9 - 8,3. |

5. Stimulierungsmittel nach Anspruch 1 zur Verwendung in einem Verfahren der Stimulierung von Lymphdrainage bei der Behandlung von Patienten mit chronischen Hauterkrankungen, nichtonkologischen Erkrankungen des Verdauungstrakts und chronischer obstruktiver Bronchitis wobei das Mittel 30 - 40 Minuten vor und 2 - 2,5 Stunden nach jeder Mahlzeit bei insgesamt 1,5 - 2,5 1 täglich eingenommen wird, und die erste Dosis auf leerem Magen eingenommen wird.

## Revendications

1. Agent pour stimuler le drainage lymphatique, **caractérisé en ce qu'**il contient de l'eau potable qui a été soumise à un traitement à l'aide d'une baromembrane et qui possède un potentiel redox de +200 à +343 mV, une teneur totale en minéraux de 25 à 130 mg/l et un indice de pH de 6,9 à 8,3.

2. Agent pour stimuler le drainage lymphatique selon la revendication 1, **caractérisé en ce que** la dose quotidienne s'élève de 1,5 à 2,5 l.

3. Agent pour stimuler le drainage lymphatique selon la revendication 1, que l'on peut utiliser dans la thérapie complexe de maladies cutanées chroniques, de maladies non oncologiques du système gastro-intestinal et de la bronchite chronique obstructive.

4. Procédé pour obtenir un agent destiné à stimuler le drainage lymphatique, **caractérisé en ce qu'**on amène d'abord l'eau à l'état d'eau potable et on la traite ensuite dans une installation faisant appel à une baromembrane, en faisant passer l'eau à travers des membranes constituées d'une matière semi-perméable avec une dimension des trous de 0,0001 à 0,005 µm, l'eau purifiée étant saturée avec des macroéléments et des oligo-éléments et possédant les caractéristiques suivantes :
potentiel redox de + 200 à +343 mV ;
teneur totale en minéraux de 25 à 130 mg/l
indice de pH de 6,9 à 8,3.

5. Agent pour stimuler le drainage lymphatique selon la revendication 1, à utiliser dans un procédé de stimulation du drainage lymphatique dans le traitement médical de patients souffrant de maladies cutanées chroniques, de maladies non oncologiques du système gastro-intestinal et de bronchite chronique obstructive, et dans lequel l'agent est pris 30 à 40 minutes avant et 2 à 2,5 heures après chaque repas, à concurrence de 1,5 à 2,5 l par jour, la première dose étant prise le matin à jeun.
